# EUROPEAN PATENT APPLICATION

(11) **EP 4 821 435 A1**
(43) Date of publication of application: **14.10.2026**
(21) Application number: 25169855.1
(22) Date of filing: 10.04.2025
(51) Int. Cl.: A61B 5/259, A61B 5/273

(54) **METHOD FOR MANUFACTURING AN ARRANGEMENT COMPRISING AN ELECTRODE FOR ATTACHMENT TO THE HUMAN SKIN**

(71) Applicant: Leonhard Lang GmbH, 6020 Innsbruck (AT)
(72) Inventor: LANG, Burrhus, 6020 Innsbruck (AT)
(74) Representative: Torggler & Hofmann Patentanwälte - Innsbruck

(57) **Abstract**

Method for manufacturing an arrangement (1) comprising an electrode (2) for attachment to the human skin and at least one cable (3) connected to the electrode, with the following steps:
- Providing a conducting element (4), preferably a conducting composite foil, comprising at least one electrically non-conductive carrier (5) and at least one electrically conductive conductor (6), wherein the conductor (6) is arranged at the carrier (5) and covers the carrier (5) at least in certain areas,
- Removing the carrier (5) in at least one defined area (5a) by means of a machining process, preferably milling and/or laser ablation, so that an opening (7) for contacting the conductor (6) through the carrier (5) is provided in the carrier (5), and
- Attaching a cable (3) to the conductor (6) in the opening so that the cable (3) is electrically connected to the conductor (6).

## Description

The present invention concerns a method for manufacturing an arrangement comprising an electrode for attachment to the human skin and at least one cable connected to the electrode as well as an arrangement comprising an electrode for attachment to the human skin and at least one cable connected to the electrode, preferably manufactured by said method.

Such arrangements are well known from the prior art. Electrodes in such arrangements are usually medical electrodes. They can be used as measuring electrodes to derive electrical signals from the human body or as therapy electrodes to supply electricity to the human body. For this purpose, the electrodes are attached to the skin and generally have an electrically conductive gel or another electrical contact medium on their underside, which is in electrically conductive contact with a conductor of the electrode. A cable can be electrically connected to the conductor, via which currents can be derived from the electrode or fed to the electrode.

In the state of the art, a multitude of options for connecting the cable to the conductor are known. Most commonly, a connecting element is provided for connecting the cable to the conductor. Such a connecting element connects the cable with a front side of the conductor, the front side facing the skin of a patient. Connecting elements usually make use of some variation of a riveting process to connect the cable to the conductor.

It is also known to solder the cable to said underside of the conductor.

These options for connecting the cable have several drawbacks. First a complex process for riveting is necessary, as riveting takes place in an area later covered with gel and which has to be insulated in order to prevent high local current concentrations. Second, a complex process is needed to provide a gel-free area on the conductor. This gel-free area also has to be insulated. Third, the electrode has to be widened to create a lateral area for the cable connection, wherein the lateral area also has to be insulated.

When the conductor is in the form of an electrically conductive plastic foil, e.g. a carbon-doted PVC foil, it is known to connect the cable to a back side of the conductor, which faces away from the skin toward the carrier. Thereto, the strands of the cable are fanned out and glued to the back side of the conductor.

Again, such an embodiment has some disadvantages. First, the resistance of the conductive plastic foil leads to an uneven current distribution over the surface of the electrode. Second, handling of the electrode in production can pose problems such as handling of a support foil or paper for the conductive plastic foil, the temperature resistance of such foils or solvent resistance of the conductive plastic foil or its support foil or paper. Third, such electrodes are rather costly.

The objective technical problem to be solved by the invention is to provide an improved method as well as an improved arrangement, in which the disadvantages of the prior art are at least partially avoided.

This problem is solved by a method according to claim 1 and an arrangement according to claim 11.

According to the invention, at least the following steps are provided with a method for manufacturing an arrangement comprising an electrode for attachment to the human skin and at least one cable connected to the electrode as mentioned above:
- Providing a conducting element, preferably a conducting composite foil, comprising at least one electrically non-conductive carrier and at least one electrically conductive conductor, wherein the conductor is arranged at the carrier and covers the carrier at least in certain areas,
- Removing the carrier in at least one defined area by means of a machining process, preferably milling and/or laser ablation, so that an opening for contacting the conductor through the carrier is provided in the carrier, and
- Attaching a cable to the conductor in the opening so that the cable is electrically connected to the conductor.

Due to the carrier being removed from the conductor, it is possible to connect the cable to the back side of the conductor in an easy manner and without the need for an additional connecting element. This goes along with a variety of advantages.

First, a homogenous current distribution can be achieved at the front side of the conductor. Second, the process for attaching the cable to the back side of the conductor does not require any complex steps, such as fanning the strands of a cable, riveting or insulation steps.

Furthermore, the costs for producing an electrode can be reduced as there is no additional material (like a connecting element) and fewer process steps needed. Therein, even small savings regarding material and/or process steps can result in a large cost reduction, as medical electrodes are mass-produced products that are manufactured in very large quantities.

Also, new electrode geometries are possible, since the front side of the conductor is not needed for connecting the cable to the conductor.

A machining process may refer to a traditional machining process, where a material is removed by the removal of chips via at least one (defined or undefined) cutting edge, e.g**.** milling. However, a machining process may also refer to the removal of material via a laser, e.g**.** laser ablation.

Regarding an electrode according to the invention, it is provided that the electrode comprises a conducting element, preferably a conducting composite foil, comprising at least one electrically non-conductive carrier and at least one electrically conductive conductor, wherein the conductor is arranged at the carrier and covers the carrier at least in certain areas, wherein an opening for contacting the conductor through the carrier is provided in the carrier, and that a cable is electrically connected to the conductor in said opening.

Further advantageous embodiments of the invention are defined in the dependent claims.

It may be provided that the conductor is connected to the carrier by means of a bonding agent, preferably an adhesive or a thermoplastic, wherein a bonding agent layer formed by the bonding agent between the conductor and the carrier is removed when the conductor is removed by means of the machining process.

By removing the bonding agent a better electrical connection between the cable and the conductor can be achieved.

A bonding agent is a simple way of connecting the conductor to the carrier. In this case, the conductor is bonded to the carrier.

According to a further embodiment of the invention, the chips produced during the removal of the carrier and/or bonding agent layer are sucked off.

This is an easy way to remove the resulting chips. No additional equipment is required, as would be the case when removing punched parts, for example.

It can also be provided that in a further step an electrically conductive contact medium is applied to the conductor.

For this purpose, the contact medium can either be provided in the form of a prefabricated web and applied to the conductor and/or the carrier, for example. It is also possible that the contact medium is available in liquid form and is poured onto the conductor and/or the carrier and then cured. Even after curing, the contact medium can still have a gel-like consistency, for example.

In an advantageous embodiment a cover element is applied to the contact medium in a further step. Preferably, the cover element can again be designed as a foil.

Such a cover element can protect the contact medium before the electrode is applied to the skin.

It may be provided that in a further step the carrier and/or the contact medium is cut through, preferably by means of a punching process.

Furthermore, it can also be provided that in a further step the cover element is cut through, preferably by means of a punching process.

This allows the electrode to be finally formed. In a preferred embodiment, the carrier and the contact medium are cut through together. In this case, the cover element remains intact so that the individual electrodes are arranged together on the cover element.

In a preferred embodiment, the cable can be attached to the conductor in the opening by means of material bonding, in particular by adhesive bonding and/or soldering.

If the cable is attached to the conductor by adhesive bonding, it may be provided that an electrically conductive adhesive is used.

It may be provided that the electrically conductive adhesive is only conductive in one specific direction, e.g. in a direction orthogonal to the sheet material.

It may be provided that the mechanical attachment of the cable is provided at a different location and/or at the same location as the electrical attachment.

In particular, the cable can be electrically connected to the conductor and mechanically connected to the conductor and/or the carrier.

The means for electrically connecting the cable and the means for mechanically connecting the cable can be the same or different means.

The features mentioned above allow, together or distinct from each other, to provide the most suitable configuration for connecting the cable to the electrode depending on the specific electrode and its use case.

According to another preferred embodiment, the cable can be a flexible flat cable.

A flexible flat cable is both flat and flexible, with flat solid conductors.

Using a flexible flat cable in connection with an electrode results in numerous advantages over commonly used cables.

Commonly used cables often comprises at least one metal strand with a circular cross-section, which is encased in an insulation. Also, multiple strands, sometimes twisted together, are common. Alternatively, cables with strands made from conductive plastics are known. In some cases, cables with a conductor printed onto insulating material are used. Such conductors can be printed from ink comprising metal or conductive plastic particles. The conductor may be sintered after printing.

With respect to cables with strands, the amount of materials needed to produce a flexible flat cable with a similar internal resistance can be reduced significantly. Thus, sustainability of single-use arrangements with flexible flat cables is much higher when compared to cables used in the prior art. Also, costs for disposal can be reduced. Additionally, the cost for manufacturing as well as transporting such arrangements are lower due to reduced material expenses and a reduced weight.

With respect to printed cables, a flexible flat cable fulfils the requirements regarding their electrical properties, especially internal resistance, while still using less material, as discussed above.

Furthermore, a desired cross-section of the cable can be easily achieved by selecting an appropriately thick sheet material and adjusting the width of the sheet material. Also, a flexible flat cable can be easily and directly bonded to the conductor of the electrode, foregoing the need for additional connecting elements or complex process for connecting the cable, such as riveting.

Although a person skilled in the art is well aware of meaning of the term "flexible flat cable", it shall briefly be described how a flexible flat cable differs from cables in the prior art.

With respect to common cables with strands, the cables as well as their cable conductors (the strands) are of a circular cross section. Flexible flat cables are, as by their name, flat. However, not only the flexible flat cable itself is flat but also the cable conductor of the flexible flat cable (the sheet material). This distinguishes flexible flat cable from ribbon cables, with flexible flat cables being thinner than ribbon cables. Thus, a sheet material is an element whose thickness is small compared to its other dimensions.

Printed (or sputtered) cables may also be formed in a flat manner as described above. However, a printed cable conductor of a cable is not comparable to a sheet material which serves as cable conductor of flexible flat cables.

A sheet material differs from a printed cable conductor, inter alia, thereby that the sheet material is structurally integral by itself. Meaning, no base material or the like is needed to support the sheet material. In other words, the sheet material is self-supporting.

In contrast, a printed (or sputtered) cable conductor necessarily needs some form of base material onto which the cable conductor is printed. Also, printed cable conductors usually are less flexible than a sheet material due to their different material properties.

It can further be provided that the conductor and/or the sheet material comprises at least one metal, preferably is made of at least one metal.

Preferably, the carrier as well as the conductor are provided in form of a foil, so that the conducting element is a conducting composite foil.

The conductor is electrically conductive and can preferably be made of a metal.

The carrier consists of at least one electrically non-conductive, i.e**.** electrically insulating, material, for example a plastic such as polyester or polysterol. Other electrically insulating materials are also conceivable

With respect to an arrangement according to the invention, it may be provided that the cable is connected to the conductor in the opening by means of material bonding, in particular by adhesive bonding and/or soldering.

Furthermore, the cable can preferably be a flexible flat cable.

In a further embodiment the conductor comprises at least one metal, preferably is made of at least one metal.

Furthermore, it may be provided that an electrically conductive contact medium is arranged on the carrier and/or at least partly on the conductor.

In a preferred embodiment, the cable may comprise a stabilizing element, wherein the stabilizing element defines a minimum admissible bending radius of the cable.

Thus, snapping of the cable can be prevented.

It may be provided that the stabilizing element consists of at least one fiber arranged at or within the cable.

This is an easy and cost-effective way to realize a stabilizing element.

The contact medium is used to make electrical contact with the skin. The contact medium can be in the form of a conductive gel or a conductive adhesive, for example.

It may also be provided that a cover element is arranged on the contact medium. The cover element can protect the contact medium from damage and soiling.

The covering element can be designed as an abhesive film, for example. This allows for an easy removal of the covering element before using the electrode.

In a further embodiment, it may be provided that an impedance layer is arranged at least partly on the conductor.

The impedance layer can also be electrically conductive. By partially removing the impedance layer from the conductor, the current output from the electrode to the human skin can be altered

It may also be provided that a plaster layer extending beyond the carrier is arranged on the carrier. The plaster layer is arranged on a side of the carrier on which no conductor is arranged.

The plaster layer can be used to achieve a more secure hold of the electrode on the skin.

Further details and advantages of preferred embodiments of the invention are explained in more detail below with reference to the drawings. Therein shows
- Fig. 1: a first step of a method for manufacturing an arrangement,
- Fig. 2A-F: different further steps of a method for manufacturing the arrangement,
- Fig. 3-8: further steps of a method for manufacturing the arrangement,
- Fig. 9A-B: schematic views of an electrode,
- Fig. 10A-E: schematic views of different embodiments of a cable,
- Fig. 11A-B: schematic views of the arrangement,
- Fig. 12: a schematic view of an embodiment of an arrangement according to the prior art,
- Fig. 13A-B: schematic views of an embodiment of an electrode according to the invention and according to the prior art,
- Fig. 14A: a cross-sectional view of an embodiment of a flat cable with a printed conductor according to the prior art, and
- Fig. 14B: a cross-sectional view of an embodiment of a flexible flat cable.

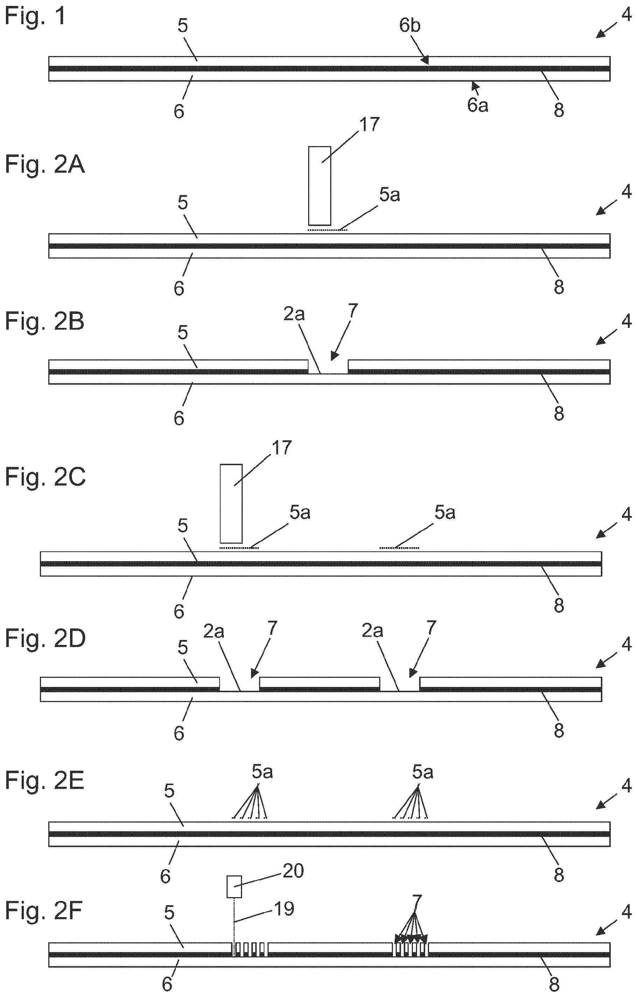

From Figs. 1 to 8, a preferred embodiment of a method for manufacturing of an embodiment of an arrangement 1 can be seen. The Figs. 1 to 8 thereto provide a schematic view of a cross-section of an arrangement 1 at different points during a manufacturing process.

In a first step, a conducting element 4 is provided. The conducting element 4 comprises an electrically non-conductive carrier 5 and an electrically conductive conductor 6. The carrier 5 and the conductor 6 are bonded together by a bonding agent, the bonding agent forming a bonding agent layer 8.

In the present embodiment, the conducting element 4 thus is a conducting composite foil.

The carrier 5 may be made of a plastic, for example from such as polyester or polysterol. The conductor 6 may comprise at least one metal and preferably is made from at least one metal. However, the conductor may also be made from a conductive plastic foil.

The conductor 6 may at least partly be covered by a suitable redox-pair for depolarizing the electrode 2.

The conductor 6 comprises a front side 6a, facing toward the skin of a patient, and a back side 6b, facing toward the carrier 5 and thus away from the skin of a patient.

The front side 6a and back side 6b are thus substantially opposite as well as parallel to each other.

Figs. 2A and 2B, 2C and 2D as well as 2E and 2F disclose different embodiments of a further step for manufacturing an arrangement 1.

It can be seen that a defined area 5a of the carrier 5 is set (dotted lines), wherein the carrier 5 is subsequently removed in the defined areas 5a. This is achieved via machining, in particular via milling with a milling cutter 17. The bonding agent layer 8 is also removed in the defined areas 5a during this process, exposing the conductor 6 on the back side 6b in said defined areas 5a.

Via the machined openings 7, the conductor 6 can be accessed on the back side 6b to connect a cable 3 to the conductor 6. A contact area 2a is thus provided at the back side of the conductor 6 in the opening 7.

In Figs. 2A and 2B, only one opening 7 is produced, whereas two openings 7 are provided in Figs. 2C and 2D. In Fig. 2E and 2F, multiple slit-like openings 7 are produced. Such slit-like openings 7 may be produce via machining with a laser beam 19 produced by a laser 20.

With the latter, strands of a cable 3 could be easily and securely attached in the slit-like openings 7.

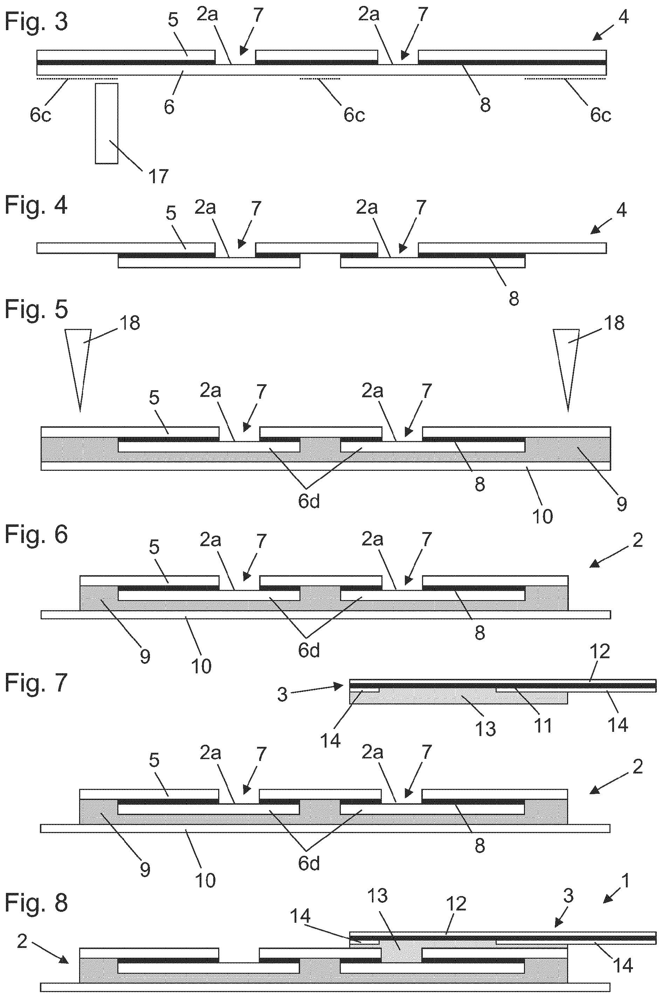

Fig. 3 shows a further step for manufacturing of an arrangement 1, on basis of the steps shown in Figs. 2C and 2D. On the conductor 6, defined areas 6c are set and subsequently the conductor 6 is removed in said defined areas 6c. This is achieved via machining, in particular via milling with a milling cutter 17. The milling cutter 17 may be the same or a different milling cutter 17 than in the previous steps.

Again, the bonding agent layer 8 is removed in the defined areas 6c during this process. However, the bonding agent layer 8 may also remain in the defined areas 6c.

Thereby, two conductor sections 6d are produced, the conductor sections 6d being separate from each other. In particular, the conductor sections 6d are electrically isolated from each other. This can be seen from Fig. 4 as well as Figs. 9A, 9B, 12A, 12B or 13A for example.

In a further step, a contact medium 9 is applied onto the conductor sections 6d as well as the space around the conductor sections 6d.

In a further step, a cover element 10 is applied to the contact medium 9.

Fig. 5 shows the state of the arrangement 1 after the aforementioned steps. In Fig. 5, punching knives 18 can be seen. By means of the punching knives 18, the carrier 5, and, if applicable, the conductor 6, and the contact medium 9 can be cut to define the shape of an electrode 2.

Alternatively or additionally to the punching knives 18, other types of knives may be provided, for example kiss cutting knives.

The cover element 10 is not cut in the figure shown. However, the cover element 10 may also be cut in the same or a different shape by the same or different punching knives 18.

After cutting, an electrode 2 is present, as seen in Fig. 6.

In a further step, a cable 3 is provided. In the present embodiment, the cable 3 is a flexible flat cable with an electrically conductive sheet material 11. The sheet material 11 is arranged on a flat, flexible and electrically non-conductive base layer 14.

Further, a flat, flexible and electrically non-conductive top layer 12 is provided, wherein the sheet material 11 is electrically insulated from the environment by means of the top layer 12 and the base layer 14.

The sheet material 11 is also flat and flexible. In the present embodiment, the sheet material 11 is a sheet metal, for example produced by a rolling process.

An opening 14a is provided in the base layer 14, wherein the sheet material 11 is accessible through the opening 14a.

In the present embodiment, an adhesive 13 is provided with cable 3. The adhesive 13 is provided in the opening 14a on the sheet material 11 as well as on parts of the base layer 14. The adhesive 13 an electrically conductive adhesive 13.

The adhesive 13 may additionally or alternatively be provided on the electrode 2.

In a further step, the cable 13 is connected to the conductor 6 in the contact area 2a in the opening 7 by means of the electrically conductive adhesive 13. Thus, an electric contact is established between the conductor 6 and the sheet material 11 by means of the electrically conductive adhesive 13.

Further, a mechanical connection between the cable 3 and the electrode 2 is achieved via the adhesive 13.

In this embodiment, the sheet material 11 is connected to the connector 6 as well as the carrier 5 via the adhesive 13. The base layer 14 is connected to the carrier 5.

It should be noted that the above-mentioned steps may also be performed in a different order and/or at least some of the steps may be performed parallel to each other.

Also, not all steps are absolutely necessary. It may be provided that only some of the aforementioned steps are performed to manufacture an arrangement.

Especially, it may be provided that the steps involving the machining of the conductor 6 are omitted.

It may be provided that the electrode 2 is not manufactured from scratch but rather directly provided. The electrode 2 provided may therein also be manufactured with a different process to the one described above. The carrier 5 of the provided electrode 2 may than be machined as described.

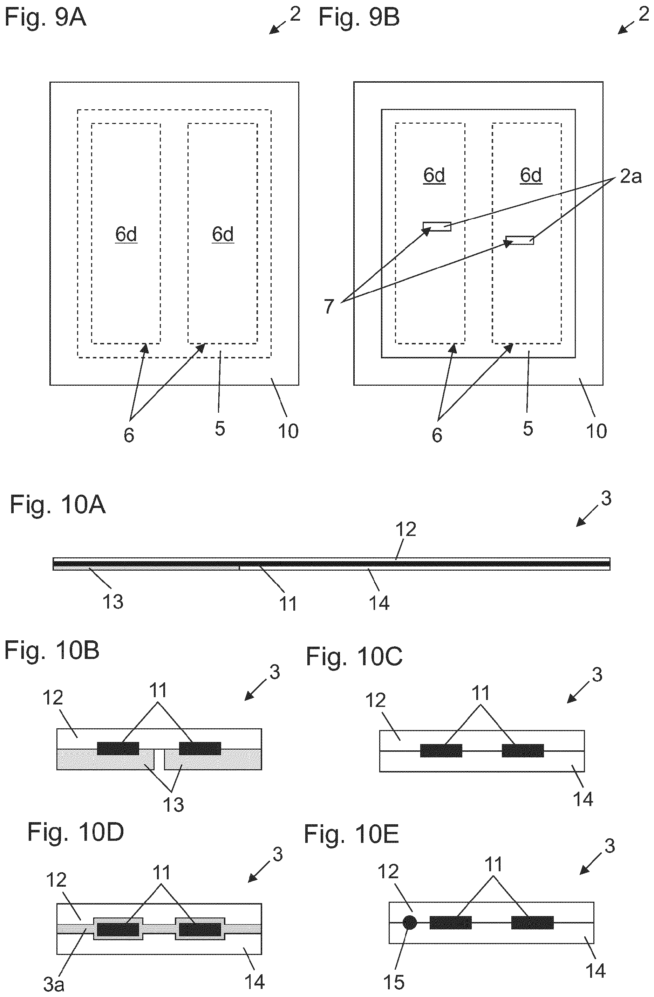

Fig. 9A shows a schematic view on a patient side of an electrode 2, facing toward the patient when in use. Fig. 9B shows a schematic view on an opposite side of an electrode 2, facing away from the patient when in use.

The dashed lines indicate that the corresponding element is hidden underneath another element, as is common in technical drawings.

It can be seen that the electrode 2 comprises a conductor 6 with two conductor sections 6d. The conductor sections 6d are separate from each other and in particular electrically insulated from each other.

The conductor 6 is arrange on a carrier 5. A contact medium 9 is applied on the conductor 6 and the carrier 5, with a cover element 10 arranged thereon. The cover element 10 extends beyond the rest of the electrode 2, which allows for an easier removal of the cover element 10.

It can be seen that the electrode 2 comprises two openings 7 in the carrier 5, wherein the openings 7 are displaced in a longitudinal direction as well as a transversal direction of the electrode 2.

Fig. 10A shows a length-wise cross-section of cable 3. Again, the cable 3 is a flexible flat cable. In contrast to the cable 3 shown in Figs. 7 and 8, the opening 14a in the base layer 14 extends to an end of the cable 3.

In this embodiment, an electrically conductive adhesive 13 can be provided in the opening 14a.

Fig. 10B shows a transversal cross section of the cable 3 in an area of the opening 14a and Fig. 10C in any other area.

It can be seen that two patches of adhesive 13 are provided, each patch being arranged on one of the sheet materials 11.

The patches of adhesive 13 are separated from each other to avoid a short circuit.

However, if an electrically conductive adhesive is provided, which is only conductive in a direction orthogonal to the sheet materials 11, only one patch of adhesive covering the whole cross-section of the cable 3 may be provided.

It can be seen that two flat sheet materials 11 are provided between the top layer 12 and the base layer 14. The sheet materials 11 each have a rectangular cross-section.

In Fig. 10D a bonding agent 3a which bonds the parts of cable 3 together is highlighted.

Fig. 10E shows a further embodiment of a cable 3. The cable 3 additionally comprises a stabilizing element 15 in form of at least one fiber arranged at or within the cable 3. The fibre may be made of nylon or the like.

Such a stabilizing element 15 defines a minimum admissible bending radius of the cable 3, thus preventing snapping of the cable 3, in particular the sheet material 11, due to bending of the cable 3.

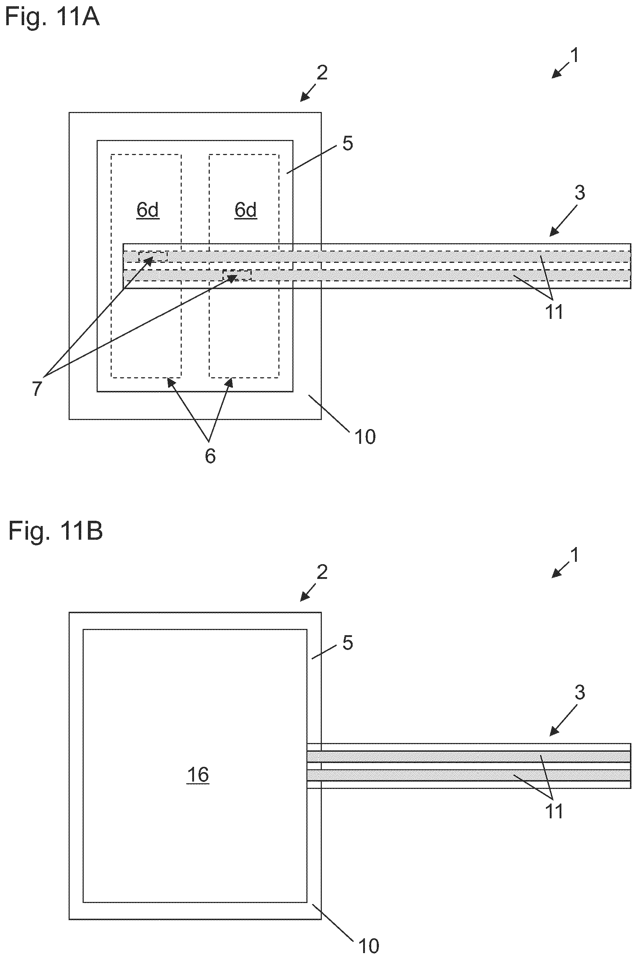

Figs. 11A and 11B show a schematic view on a side of an arrangement 1 facing away from the patient when in use.

It can be seen that the two sheet materials 11 of the cable 3 line up with the two openings 7 in the carrier 5 of the electrode 6. Each of the sheet materials 11 is thus electrically connected with one of the two conductor sections 6.

In Fig. 11B an additional plaster layer 16 is arranged on the carrier 5. This plaster layer 16 can be, exclusively or additionally, be used to fix the arrangement 1 on the skin of a patient. Further, the plaster layer 16 additionally fixes the cable 3 to the electrode 2, increasing the stability of the connection between electrode 2 and cable 3.

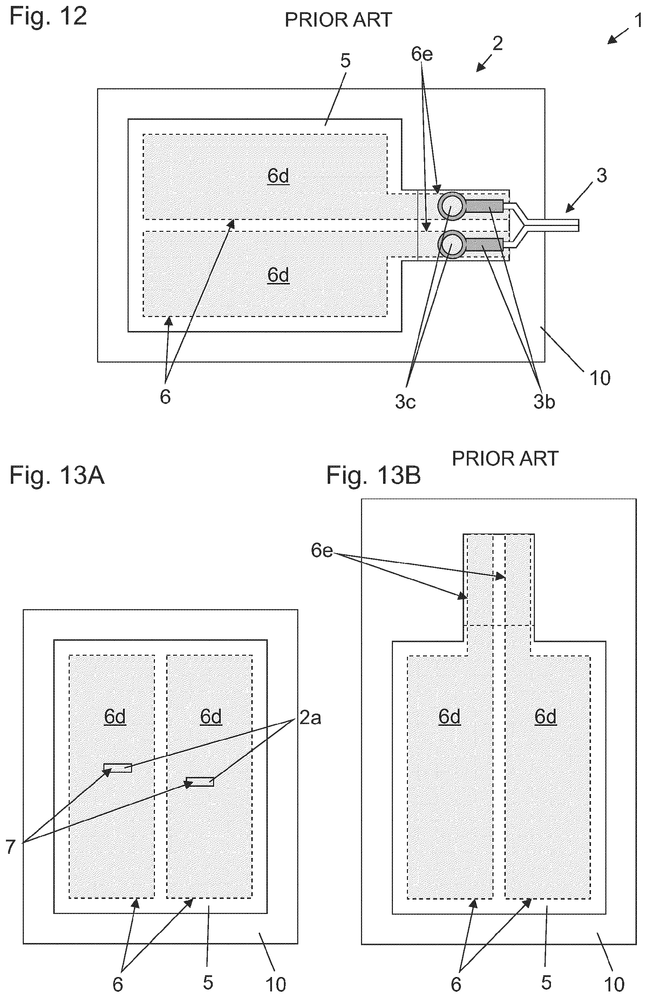

Fig. 12 shows a schematic view of an embodiment of an arrangement 1 according to the prior art. It can be seen that extensions 6c of the conductor sections 6d are provided on the carrier 5, the extension 6e extending beyond the contact medium 9. The contact areas 2a are provided on the extensions 6e. A cable 3 comprises cable lugs 3b, which are riveted to the extensions 6c to connect the cable to the conductor 6 and the electrode 2.

Such an embodiment according to the prior art uses much more material than an arrangement according to the invention. This can also be seen from Figs. 13A and 13B, where schematic views of an embodiment of an electrode 2 according to the invention and according to the prior art are shown next to each other.

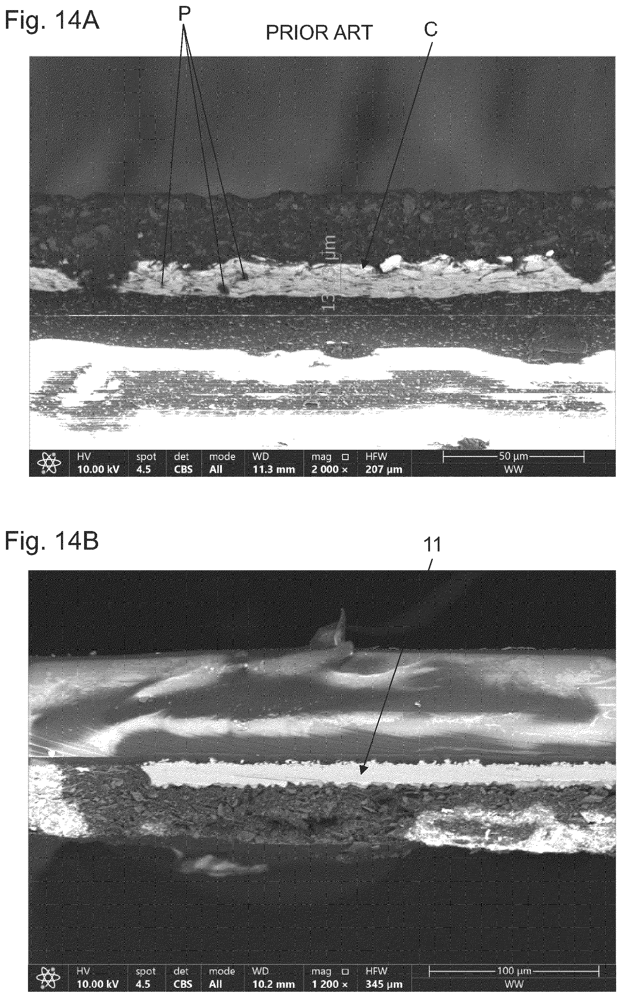

Fig. 14A shows a cross-sectional view of an embodiment of a flat cable 3 with a printed conductor C, and Fig. 14B a cross-sectional view of an embodiment of a flexible flat cable 3.

From these figures the difference between a flat, printed cable 3, as known from the prior art to a flexible flat cable 3 as used with the invention can be clearly seen.

The printed conductor C shows a multitude of pores P enclosed therein, which are a result of the printing process. As described above, such pores P have a significant negative impact on the electrical properties of the conductor C.

As can be seen from Fig. 14B, the sheet material 11 of the flexible flat cable 3 shows a clean metallic cross-section without pores P. This can be achived by using a sheet metal for example.

### List of reference signs:

- 1: Arrangement
- 2: Electrode
2a contact area
- 3: Cable
3a bonding agent
3b cable lug
3c rivet
- 4: Conducting element
- 5: Carrier
5a defined area
- 6: Conductor
6a front side
6b back side
6c defined area
6d conductor section
6e extension
- 7: Opening
- 8: Bonding agent layer
- 9: Contact medium
- 10: Cover element
- 11: Sheet material
- 12: Top layer
- 13: Adhesive
- 14: Base layer
14a opening
- 15: Stabilizing element
- 16: Plaster layer
- 17: Milling cutter
- 18: Punching knife
- C: printed conductor
- P: pores

## Claims

1. Method for manufacturing an arrangement (1) comprising an electrode (2) for attachment to the human skin and at least one cable (3) connected to the electrode, **characterized by** the following steps:
- Providing a conducting element (4), preferably a conducting composite foil, comprising at least one electrically non-conductive carrier (5) and at least one electrically conductive conductor (6), wherein the conductor (6) is arranged at the carrier (5) and covers the carrier (5) at least in certain areas,
- Removing the carrier (5) in at least one defined area (5a) by means of a machining process, preferably milling and/or laser ablation, so that an opening (7) for contacting the conductor (6) through the carrier (5) is provided in the carrier (5), and
- Attaching a cable (3) to the conductor (6) in the opening so that the cable (3) is electrically connected to the conductor (6).

2. Method according to claim 1, wherein the conductor (6) is connected to the carrier (5) by means of a bonding agent, preferably an adhesive or a thermoplastic, wherein a bonding agent layer (8) formed by the bonding agent between the conductor (6) and the carrier (5) is removed when the carrier (5) is removed by means of the machining process.

3. Method according to claim 1 or 2, wherein chips produced during the removal of the carrier (5) and/or bonding agent layer (8) are sucked off.

4. Method according to one of claims 1 to 3, wherein in a further step an electrically conductive contact medium (9) is applied to the conductor (6).

5. Method according to claim 4, wherein in a further step a cover element (10) is applied to the contact medium (9).

6. Method according to one of claims 1 to 5, wherein in a further step the carrier (5) and/or the contact medium (9) is cut through, preferably by means of a punching process.

7. Method according to claim 5, wherein in a further step the cover element (10) is cut through, preferably by means of a punching process.

8. Method according to one of claims 1 to 7, wherein the cable (3) is attached to the conductor (6) in the opening (7) by means of material bonding, in particular by adhesive bonding and/or soldering.

9. Method according to one of claims 1 to 8, wherein the cable (3) is a flexible flat cable.

10. Method according to one of claims 1 to 9, wherein the conductor (6) comprises at least one metal, preferably is made of at least one metal.

11. Arrangement (1) comprising an electrode (2) for attachment to the human skin and at least one cable (3) connected to the electrode (2), preferably manufactured by a method according to one of claims 1 to 10, wherein the electrode (2) comprises a conducting element (4), preferably a conducting composite foil, comprising at least one electrically non-conductive carrier (5) and at least one electrically conductive conductor (6), wherein the conductor (6) is arranged at the carrier (5) and covers the carrier (5) at least in certain areas, **characterized in that** an opening (7) for contacting the conductor (6) through the carrier (5) is provided in the carrier (5), and that the cable (3) is electrically connected to the conductor (6) in said opening (7).

12. Arrangement (1) according to claim 11, wherein the cable (3) is connected to the conductor (6) in the opening (7) by means of material bonding, in particular by adhesive bonding and/or soldering.

13. Arrangement (1) according to one of claims 11 or 12, wherein the cable (3) is a flexible flat cable.

14. Arrangement (1) according to one of claims 11 to 13, wherein the conductor (6) comprises at least one metal, preferably is made of at least one metal.

15. Arrangement (1) according to one of claims 11 to 14, wherein the cable (3) comprises a stabilizing element (15), wherein the stabilizing element (15) defines a minimum admissible bending radius of the cable (3), preferably wherein the stabilizing element (15) consists of at least one fibre arranged at or within the cable (3).
